# EUROPÄISCHE PATENTANMELDUNG

(11) **EP 3 779 099 A1**
(43) Veröffentlichungstag der Anmeldung: **17.02.2021**
(21) Anmeldenummer: 19191181.7
(22) Anmeldetag: 12.08.2019
(51) Int. Cl.: E04H 1/12, E04F 13/08, A61M 21/00

(54) **BETRETBARER AUFENTHALTSRAUM**

(71) Anmelder: Ungefucht, Robert, 92224 Amberg (DE)
(72) Erfinder: Ungefucht, Robert, 92224 Amberg (DE)
(74) Vertreter: Bittner, Bernhard

(57) **Zusammenfassung**

Betretbarer Aufenthaltsraum für Personen mit wenigstens einer Seitenwandung (2) und mit wenigstens einer Decke (6), wobei wenigstens an der Seitenwandung (2) oder an der Decke (6) eine Vielzahl von Stückkörpern (10) angeordnet ist, wobei sich diese Stückkörper (10) hinsichtlich ihrer äußeren Gestalt voneinander unterscheiden und wobei diese Stückkörper (10) wenigstens abschnittsweise eine nichtebene Oberflächengestalt aufweisen, wobei diese Stückkörper (10) mittels wenigstens einer Befestigungseinrichtung (4) an der Seitenwandung oder der Decke befestigt sind,
dadurch gekennzeichnet, dass
die Befestigungseinrichtung (4) ein Netzelement (42) aufweisen, welches Netzmaschen (44) aufweist, die derart dimensioniert sind dass die Stückkörper nicht durch diese Netzmaschen (44) treten können und/oder die Befestigungseinrichtung (4) ein drahtartiges Element (46) aufweist, welches durch eine durch den Stückkörper (10) hindurch verlaufende Öffnung (12) geführt ist und/oder die Stückkörper mittels eines Klebstoffes an den Seitenwand oder der Decke befestigt ist.

## Beschreibung

Die vorliegende Erfindung bezieht sich auf einen betretbaren Aufenthaltsraum und insbesondere einen mit ornativen Elementen und insbesondere Bernstein versehenen Aufenthaltsraum. Bei diesem Aufenthaltsraum kann es sich beispielsweise um ein sogenanntes Bernsteinzimmer handeln, daneben jedoch auch um eine Sauna, eine Infrarotsauna, einen Ruheraum und einen Wellnessbereich oder dergleichen. Derartige Aufenthaltsräume sind aus dem Stand der Technik seit Langem bekannt. Auch ist es teilweise bekannt, diese Räume bzw. deren Decken und Wände oder auch die Böden mit Ziergegenständen oder dekorativen Elementen zu versehen, die insbesondere auch die Optik derartiger Räume verschönern sollen. Daneben können jedoch bestimmte Ziergegenstände wie etwa Bernsteine auch der Gesundheitsvorsorge dienen.

Oftmals fällt es auch bestimmten Personen sehr schwer, sich in einem sehr ruhigen Raum völlig zu entspannen, so dass dieser Personenkreis eine Art aktiver Erholung benötigt. Eine Person, welche den Raum (Bernsteinzimmer / Sauna und andere mit der nachfolgenden Erfindung ausgestattete Aufenthaltsräume) nutzt soll derart von der Außenumgebung und seinen eigenen Gedanken und Problemen abgelenkt werden soll, so dass diese Person eine Tiefenentspannung und Erholung in Ruhe erfahren kann.

Ein derartiges Zierelement können beispielsweise Steine, Edelsteine, Schmucksteine oder dergleichen sein, die insbesondere an den Decken oder Wänden befindlich sein können. Problematisch ist bei derartigen Ziergegenständen insbesondere deren Befestigung an Wänden oder Deckenbereichen.

Der vorliegenden Erfindung liegt daher die Aufgabe zugrunde eine Anordnung derartiger Ziergegenstände an den Wänden, insbesondere auch, wenn eine hohe Zahl dieser Ziergegenstände befestigt werden soll, zu ermöglichen. Dabei soll insbesondere eine möglichst natürliche oder naturnahe Befestigung geschaffen werden. Insbesondere soll auch auf den Einsatz ggfs. Gesundheitsschädlicher Produkte verzichtet werden.

Dies wird erfindungsgemäß durch den Gegenstand des unabhängigen Patentanspruchs erreicht. Vorteilhafte Ausführungsformen und Weiterbildungen sind Gegenstand der Unteransprüche.

Ein erfindungsgemäßer betretbarer Aufenthaltsraum für Personen weist wenigstens eine Seitenwandung und/oder wenigstens eine Decke auf, wobei wenigstens an der Seitenwandung oder an der Decke eine Vielzahl von Stückkörpern angeordnet ist, und wobei sich diese Stückkörper wenigstens hinsichtlich ihrer äußeren Gestalt und/ oder ihrer Größe voneinander unterscheiden und wobei diese Stückkörper wenigstens abschnittsweise eine nicht ebene Oberflächengestalt aufweisen. Weiterhin sind diese Stückkörper mittels einer Befestigungseinrichtung an einer Seitenwandung oder einer Decke des Aufenthaltsraums befestigt.

Erfindungsgemäß weist dabei die Befestigungseinrichtung ein Netzelement auf, welches Netzmaschen aufweist, die derart dimensioniert sind, dass die Stückkörper nicht durch diese Netzmaschen hindurch treten können und/ oder die Befestigungseinrichtung weist ein drahtartiges Element auf, welches durch eine durch den Stückkörper hindurch verlaufende Öffnung geführt ist und/ oder die Stückkörper sind mittels eines Klebstoffes an der Seitenwand oder der Decke zumindest mittelbar befestigt.

Bei einer weiteren bevorzugten Ausführungsform ist der Aufenthaltsraum gegenüber Umgebungseinflüssen isoliert. So kann der Aufenthaltsraum beispielsweise schallisoliert sein. Daneben kann der Aufenthaltsraum auch gegenüber anderen Immissionen isoliert sein, wie beispielsweise gegenüber außerhalb des Raumes auftretenden Gerüchen oder aber außerhalb des Aufenthaltsraumes auftretenden Lichtquellen.

Neben den Decken oder Wänden könnten auch andere Oberflächen mit Bernstein versehen, beispielsweise verklebt sein, etwa Bodenflächen oder die Flächen von Gegenständen, die sich im Inneren des Aufenthaltsraumes befinden, etwa die Oberflächen von Sitz- oder Liegegelegenheiten.

Durch die hier beschriebenen Befestigungseinrichtungen wird dem Umstand Rechnung getragen, dass diese Stückkörper einerseits eine voneinander abweichende Oberflächengestalt haben und ggfs. auch unterschiedliche Gewichte und andererseits auch, dass diese in beliebiger Weise angeordnet werden sollen. Allerdings kann bevorzugt davon ausgegangen werden, dass die Stückkörper eine bestimmte Mindestgröße aufweisen, so dass insbesondere die Größe der Netzmaschen entsprechend gewählt werden kann.

Dabei kann dieses Netzelement an einer Decke des Aufenthaltsraumes angeordnet werden. Es wäre jedoch auch möglich, ein derartiges Netzelement an anderen Bereichen des Aufenthaltsraumes anzuordnen, etwa an einem Boden des Aufenthaltsraumes, z.B. unterhalb von Bänken, die ebenfalls in dem Aufenthaltsraum angeordnet sein können.

Bevorzugt weisen die besagten Stückkörper eine kantige und insbesondere nicht ebene Außengestalt auf. Bevorzugt weisen auch die einzelnen Stückkörper eine unregelmäßige Außengestalt auf, das heißt sie unterscheiden sich voneinander zumindest durch ihre Außengestalt.

Bei einer weiteren vorteilhaften Ausführungsform ist die erwähnte Bohrung durch den Stücckörper geradlinig. Bei einer weiteren bevorzugten Ausführungsform ist die besagte Bohrung innerhalb des Stückkörpers in einem Bereich platziert, der in einem befestigten Zustand des Stückkörpers näher an der Seitenwand als an der entsprechend gegenüberliegenden Oberfläche des Stückkörpers angeordnet ist. Auf diese Weise kann einerseits Befestigungsmaterial eingespart werden und andererseits kann auch der optische Eindruck verbessert werden, da das Befestigungsmaterial, wie etwa ein Draht, weniger sichtbar ist.

Falls als Befestigungseinrichtung ein Netz verwendet wird, ist es möglich, die Stückkörper lose in dieses Netz einzufüllen und gegebenenfalls auch in zwei oder mehreren übereinander angeordneten Schichten. Besonders bevorzugt sind die Stückkörper nicht an dem Netz bzw. an dessen Maschen befestigt.

Das Netz kann beispielsweise an der Decke oder einer Wand befestigt werden, bevorzugt jedoch nicht mit Klebstoff oder Draht. Die Befestigungsmethode richtet sich nah der Gestaltung des Aufenthaltsraums, beispielsweise einer Sauna oder eines Saunaraums

Es wäre jedoch auch möglich, insbesondere falls ein derartiges Netz zur Befestigung an Seitenwänden verwendet wird, die Stückkörper an dem Netz zu befestigen, was beispielsweise durch Klemmen erfolgen kann, aber auch durch die Verwendung von Klebstoffen. Besonders bevorzugt handelt es sich dabei um einen ökologischen Klebstoff. Besonders bevorzugt ist dieser Klebstoff frei von Epoxidharz.

Daneben kann der Aufenthaltsraum auch eine Ausgabeeinrichtung zur Ausgabe von Bernsteinsäure oder mit Bernstein gesättigter Luft aufweisen. Mit Bernstein gesättigte Luft bedeutet die "mit Bernsteinsäure und negativ geladenen Ionen gesättigte Luft, die aus Bernstein nach dem Erhitzen entsteht. Dies gilt für alle Lösungen, allerdings bevorzugt nicht für Bernsteinplatten. In einem Bernsteinzimmer ohne Heizung wird die Bernsteinsäure selbst leicht produziert.

Bei einer weiteren bevorzugten Ausführungsform sind die Stückkörper, bei denen es sich insbesondere um Bernsteinstücke handelt, zumindest teilweise aneinander anliegend angeordnet. Bei einer weiteren bevorzugten Ausführungsform sind zwischen einzelnen Stückgütern auch Zwischenräume oder Spalte vorgesehen. Aufgrund der unregelmäßigen Form der Oberflächen der Bernsteine entstehen Lücken beim Anbringen der einzelnen Bernsteine, insbesondere bei der Befestigung mittels Drahtelementen oder mittels Klebstoff.

Es wäre jedoch auch möglich, dass eine oder mehrere Bernsteinseiten geschliffen werden, um sicherzustellen, dass der Bernstein vollständig mit einer Montagefläche oder mit einem weiteren Bernstein in Berührung kommt

Als Bernsteinsorten kommen insbesondere baltischer Bernstein, blauer Bernstein und andere Bernsteine in Betracht.

Bei einer weiteren bevorzugten Ausführungsform sind die Stückkörper im Wesentlichen zufällt bzw. in zufälliger Anordnung befestigt.

Bernsteinsäure ist ein weißes Pulver, das sich in Wasser löst. Aufgrund dessen ist es möglich, die gegebene Zusammensetzung in den Raum zu sprühen. Beim Mischen mit Wasser wird die Bernsteinsäure als Dampf versprüht. Diese Lösung kann im Bernsteinraum, in Bernsteinsaunen und anderen Saunen (z. B. in Dampfsaunen oder türkischen Bädern) sowie in allen anderen Räumen, insbesondere solchen in denen ein Luftbefeuchter eingesetzt wird verwendet werden.

In diesem Luftbefeuchter kann dabei die Lösung gegossen werden und anschließend versprüht werden. Bei einer bevorzugten Ausführungsform weist der Aufenthaltsraum daher eine Luftbefeuchtungseinrichtung auf.

Eine weitere Verwendung von Bernsteinsäure ist das Mahlen von Granulaten und/oder Säurekristallen zu Pulver, um sie gleichmäßig über den Raum zu verteilen, z.B. zu sprühen. Wenn diese Partikel in die Atemwege gelangen, verteilen sich die Teilchen gleichmäßig im Körper. Bevorzugt weist der Aufenthaltsraum daher eine Einrichtung zum Sprühen auf, die wie im Bernsteinzimmer, einer Bernstein - Sauna oder einem anderen Raum installiert werden kann.

Bei einer weiteren bevorzugten Ausführungsform sind die an einer Oberfläche, z.B. einer Seitenwand oder der Decke befestigten Stückkörper und insbesondere Bernsteine in einer bestimmten Reihenfolge und/oder Anordnung angeordnet werden, um ein Bild zu erstellen. Bernstein oder auch andere Stückkörper kann in verschiedenen Farben, Größen und Verarbeitungsgraden auf die Wände oder die Decke angebracht werden.

Bei einer bevorzugten Ausführungsform handelt es sich bei den Stückkörper um Bernsteine bzw. um Bernsteinfragmente.

Bei einer weiteren bevorzugten Ausführungsform ist der Aufenthaltsraum aus einer Gruppe von Aufenthaltsräumen ausgewählt, welche Saunen, Ruheräume in Wellnessbereichen, Dampfbäder, Trockensaunen, Infrarotkabinen und dergleichen enthält. Es wird jedoch darauf hingewiesen, dass nicht alle Ausgestaltungen der Erfindung auch für Dampfbäder geeignet sind.

Bei einer weiteren bevorzugten Ausführungsform sind die Stückkörper an der Seitenwand oder der Decke unregelmäßig angeordnet. Auf diese Weise können die optischen Effekte, die von den Stückkörpern ausgehen, verbessert werden.

Bei einer weiteren bevorzugten Ausführungsform weist die Befestigungseinrichtung ein Material auf, welches aus einer Gruppe von Materialien ausgewählt ist, welche Kupfer, Silber, Gold, Platin und dergleichen enthält. Besonders bevorzugt handelt es sich bei dem Material um ein edles bzw. als Produkt unschädliches Material. Bevorzugt können als Befestigungseinrichtung bzw. als Materialien natürliche Materialien verwendet werden, die nicht gesundheitsschädlich sind oder solche, die beim Erhitzen und bei hohen Temperaturen keine giftigen Substanzen abgeben.

Daneben können auch Metalle verwendet werden, die auch bei medizintechnischen Anwendungen Verwendung finden. Bei einer bevorzugten Ausführungsform ist das Netzelement und/ oder das Drahtelement aus einem Material hergestellt, welches aus einer Gruppe von Materialien ausgewählt ist, welche Kupfer, Silber, Gold, Platin und dergleichen und insbesondere andere Metalle enthält.

Bevorzugt sind die Drahtelemente an den Seitenwänden und insbesondere an in den Seitenwänden angebrachten Öffnungen befestigt. Weiterhin können die Drahtelemente auch an dem Deckenelement bzw. an dortigen Öffnungen angebracht sein. Daneben wäre es auch möglich, dass sich eine Wandverkleidung und/ oder eine Deckenverkleidung aus einer Vielzahl von plattenartigen Körpern zusammensetzt, an denen jeweils die Stückkörper entweder wie hier ausgeführt mit den Drahtelementen oder mit den Netzelementen angeordnet sind. Diese Ausgestaltung wäre jedoch auch für andere Oberflächen, wie etwa den Boden oder die Oberflächen innerhalb des Aufenthaltsraum befindlicher Gegenstände denkbar.

So wäre es denkbar, dass beispielsweise eine perforierte Oberfläche, etwa eine Holzoberfläche zur Verfügung gestellt wird und durch diese Perforationen die Drahtelemente geführt werden. Die Drahtelemente könnten dann an der Rückseite dieser Holzoberfläche (d.h. der Rückseite bezüglich des Innenraums verdrillt werden.

Daneben ist es auch möglich, dass ein Träger, wie etwa eine Platte mit den daran angeordneten Stückkörpern neben, an auf und/oder über einem Ofen angebracht wird, insbesondere um so eine unmittelbare Erwärmung der Stückkörper zu erreichen. Dieser Träger kann je nach Raumgestaltung eine unterschiedliche Form haben.

Allgemein kann eine Halterung der Stückkörper hängend angeordnet sein, etwa herabhängend von einer Decke oder hängend oberhalb einer Erwärmungseinrichtung wie etwa eines Ofens. Die Halterung kann daher als hängende Struktur ausgebildet sein.

Auch wäre es möglich, dass eine perforierte Oberfläche vorgesehen ist und ein Heizelement hinter dieser perforierten Oberfläche angeordnet ist. Auch wäre es möglich, das Netzelement mit einem Stoffkörper und/oder einer Heizplatte hinter der perforierten Oberfläche anzuordnen.

Bei einer weiteren bevorzugten Ausführungsform sind Endabschnitte der Drahtelemente miteinander verbunden. So ist es möglich, dass ein Abschnitt des Drahtelements durch die oben erwähnte Bohrung der Stückkörper erstreckt und die beiden Endabschnitte aneinander gelegt und miteinander verbunden und insbesondere formschlüssig verbunden werden. Dies kann beispielsweise durch Verdrillen der Endabschnitte miteinander erfolgen.

Bei einer weiteren bevorzugten Ausführungsform wird ein Klebstoff auf natürlicher Basis eingesetzt.

Auf diese Weise kann auf weitere Klebstoffe, welche die Stückkörper an der Wand oder der Decke anordnen sollen, verzichtet werden. Bei einer weiteren bevorzugten Ausführungsform sind zur Befestigung eines Stückkörpers zwei Löcher oder mehrere Löcher in der Decke und/ oder Wand und/ oder Decken- und/ oder Wandverkleidung erforderlich.

Es wäre jedoch auch möglich, dass einzelne oder auch mehrere Löcher zur Befestigung mehrerer Stückkörper verwendet werden. So können beispielsweise Drahtabschnitte, die zur Befestigung zweier unterschiedlicher Stückkörper dienen durch das gleiche Loch geführt werden. Auch wäre es möglich, dass an einem Drahtelement zwei Stückkörper, beispielsweise hintereinander angeordnet sind. Auch wäre es möglich, dass durch ein Loch mehr als zwei Drähte geführt werden.

Bei einer weiteren vorteilhaften Ausführungsform kann ein Träger vorgesehen sein, der eine Vielzahl von Bohrungen und/oder Löchern zum Befestigen der Stückkörper aufweist. Diese Löcher könnten dabei beispielsweise gleichmäßig, etwa in der Art einer Matrix über diesen Träger verteilt sein. Auf diese Weise kann bei der Befestigung der Stückkörper ausgewählt werden, wie diese an den einzelnen Löchern positioniert werden. Bei einer bevorzugten Ausführungsform weisen die Löcher einen Querschnitt auf, der größer ist als 0,2mm, bevorzugt größer als 0,4mm und besonders bevorzugt größer als 0,6mm.

Bei einer weiteren bevorzugten Ausführungsform weisen die Löcher einen Querschnitt auf, der kleiner ist als 30mm, bevorzugt kleiner als 25mm, bevorzugt kleiner als 20mm und besonders bevorzugt kleiner als 15mm und besonders bevorzugt kleiner als 10mm.

Bei einer weiteren bevorzugten Ausführungsform weisen die Drahtelemente einen Durchmesser auf, der größer ist als 0,1 mm, bevorzugt größer als 0,2 mm und bevorzugt größer als 0,3 mm.

Bei einer weiteren bevorzugten Ausführungsform weisen die Drahtelemente einen Durchmesser auf, der kleiner ist als 10 mm, bevorzugt kleiner als 8 mm, bevorzugt kleiner als 6 mm, bevorzugt kleiner als 5 mm, bevorzugt kleiner als 4 mm, bevorzugt kleiner als 3 mm und besonders bevorzugt kleiner als 2 mm.

Der Anmelder hat ermittelt, dass diese Drahtgeometrien einerseits dem Erfordernis einer ausreichenden Stabilität und Reißfestigkeit genügen und andererseits auch dünn genug sind, um möglichst wenig die Optik zu beeinflussen. Daneben können die genannten Drahtquerschnitte bequem in die oben genannten Löcher eingefügt werden.

Bei einer weiteren vorteilhaften Ausführungsform ist das Netzelement an einer Decke befestigt. So kann beispielsweise das Netzelement an Eckbereichen einer Decke befestigt sein oder auch und bevorzugt an mehreren Stellen an der Decke angeschraubt oder in ähnlicher Weise befestigt sein.

Bei einer weiteren bevorzugten Ausführungsform weist der Aufenthaltsraum eine Belüftungseinrichtung auf. Dabei kann insbesondere neben einer an sich vorhandenen Belüftungseinrichtung je nach der Funktion des Raums eine weitere Belüftungseinrichtung vorgesehen sein, welche insbesondere den Stückkörpern dient. Damit Bernstein seine nützlichen Eigenschaften in der richtigen Weise zur Verfügung stellen kann, ist es vorteilhaft, wenn der Aufenthaltsraum nach jedem Aufenthalt von Personen, beispielsweise nach jeder Prophylaxesitzung gelüftet wird. Zu diesem Zweck ist eine zusätzliche Belüftung vorgesehen, welche angesammelte verbrauchte Luft aus dem Raum entfernt und den Aufenthaltsraum mit frischer Luft befüllt. Wenn der Bernsteinraum bzw. Aufenthaltsraum nicht benutzt wird, sollte der Bernstein möglichst trocken sein, um seine vorteilhaften Eigenschaften zu entfalten.

Bei einer weiteren vorteilhaften Ausführungsform weist die Vorrichtung eine Trocknungseinrichtung zum Trocknen der in dem Aufenthaltsraum befindlichen Luft auf.

Bei einer weiteren vorteilhaften Ausführungsform weist der Aufenthaltsraum eine Beleuchtungseinrichtung zum Beleuchten der Stückkörper auf. Dabei ist es möglich, dass derartige Beleuchtungseinrichtungen bzw. Lichtquellen bezüglich des Aufenthaltsraums hinter den Stückkörpern angeordnet sind und diese insoweit im Durchlichtverfahren beleuchten.

Es wären jedoch auch Beleuchtungseinrichtungen denkbar, welche die Stückkörper im Auflichtverfahren beleuchten, sodass diese Beleuchtungseinrichtungen auch innerhalb des Aufnahmeraums beleuchtet sein können und die Stückkörper entsprechend leuchten und/oder Licht reflektieren. Bei einer weiteren vorteilhaften Ausführungsform weist der Aufenthaltsraum einen Boden auf und bevorzugt sind auch an diesem Boden Stückkörper angeordnet. Dabei können hier die Stückkörper mittels eines Klebstoffs befestigt sein. Daneben könnte auch hier eine Befestigung der oben beschriebenen Art vorliegen.

Bei einer weiteren bevorzugten Ausführungsform weist der Aufenthaltsraum eine Heizeinrichtung zum Erwärmen des Aufenthaltsraums auf. Dabei kann es sich insbesondere um einen Elektroofen und insbesondere einen Infrarotofen und/oder einen oder mehrere Infrarotstrahler handeln. Es sind jedoch auch andere Arten einer Raumheizung denkbar, wie etwa Heizkörper, eine Fußbodenheizung oder dergleichen.

Neben oder anstelle von Infrarotstrahler und Elektroöfen können auch eine oder mehrere Heizplatten verwendet werden, welche den Bernstein langsam aufheizt aber nicht verbrennt. Derartige Heizplatten können bei allen hier beschriebenen Ausführungsformen verwendet werden.

Bevorzugt sind diese Heizplatten auch einstellbar, insbesondere hinsichtlich ihrer Leistung und ihrer Temperatur.

Falls ein mit Bernsteinen gefülltes Gitter verwendet wird, ist es möglich, dass Gitter selbst zu erwärmen oder aber eine Heizplatte einzusetzen. Diese Lösungen können, wie auch die anderen hier beschriebenen Lösungen in verschiedenen Saunatypen, Bernsteinräumen oder auch anderen belüfteten Räumen verwendet werden.

Bei einer weiteren bevorzugten Ausführungsform sind innerhalb des Raumes plattenartige Verkleidungselemente vorgesehen, wobei diese plattenartigen Verkleidungselemente bevorzugt aus oder unter Verwendung von Bernstein gefertigt sind. Bei einer bevorzugten Ausführungsform handelt es sich bei den Bernsteinen um baltische Bernsteine und/ oder blaue Bernsteine. Es kommen jedoch auch andere Bernsteinsorten in Betracht. Die Anmelderin hat ermittelt, dass mittels dieser Bernsteine eine besonders angenehme und beruhigende optische Wirkung erzielt werden kann.

Bevorzugt sind Mittel vorgesehen, welche die Luft innerhalb des Aufenthaltsraums mit Bernstein sättigen können. Dies bedeutet, dass die Luft mit Bernsteinsäure gesättigt wird, wobei vorteilhafte diese Bernsteinsäure aus Bernstein nach dem Erhitzen entsteht. Dabei kann dieses Erhitzen vorteilhaft durch eine zusätzliche Erwärmungseinrichtung bzw. Heizeinrichtung erfolgen, es ist jedoch auch möglich, dass auch ohne eine derartige Heizeinrichtung Bernsteinsäure von selbst freigesetzt wird und sich innerhalb des Aufenthaltsraums sammelt.

Bei Bernsteinsäure handelt es sich um ein weißes Pulver, das in Wasser lösbar ist bzw. das sich in Wasser löst. Aufgrund dessen ist es möglich, die ergebene Zusammensetzung in den Raum zu sprühen. Beim Mischen mit Wasser wird Bernsteinsäure als Dampf versprüht. Weiterhin ist es möglich, Bernsteinsäure durch das Mahlen oder als Mahlen von Granulaten oder Säurekristallen zu Staub zu verwenden, um sie gleichmäßig über den Raum zu sprühen. Bei einer weiteren bevorzugten Ausführungsform weist die Vorrichtung daher eine Beaufschlagungseinrichtung auf, um den Aufenthaltsraum mit Bernsteinsäure zu beaufschlagen. Falls derartige Kristalle in die Atemwege gelangen, werden sie gleichmäßig im Körper verteilt. Eine Methode zum Sprühen kann man neben dem hier beschriebenen Aufenthaltsraum auch in Saunen oder anderen Räumen verwenden.

Für die Anordnung der Stückkörper und insbesondere der Bernsteinstücke bzw. der Bernsteine sind mehrere Möglichkeiten denkbar. So kann Bernstein in verschiedenen Größen und Farbtönen und/ oder in Form von flachen oder reliefartigen Oberflächen, beispielsweise in Form einer festen gleichmäßigen Beschichtung oder eines Musters, aufgebracht werden, um so eine entsprechende Fläche zu bedecken. Das gleiche Verfahren der Oberflächenbeschichtung kann auch zur Abdeckung einer gesamten Oberfläche oder einzelnen Teile verwendet werden.

Vorteilhaft handelt es sich bei den mit den Stückkörpern bzw. mit den Bernsteinen zu besehenden Oberflächen um Holzoberflächen. Daneben wäre es auch möglich, die Stückkörper auf Glas oder anderen, insbesondere natürlichen Materialien anzubringen.

Eine Beschichtung mit Glas kann beispielsweise für Wände, Decken oder einzelne Innenteile verwendet werden, insbesondere solche, die nicht unter Druck stehen. Durch die Verwendung von Glas kann die mit Bernstein bedeckte Oberfläche hervorgehoben werden. Daneben ist so auch eine Beleuchtung der Oberfläche und/oder der Bernsteine möglich.

Bei einer Lösung in der Beschichtung von Holzoberflächen mit Bernstein ist es auch möglich und bevorzugt, eine Hintergrundbeleuchtung zu verwenden und die erforderlichen Lichtmuster bzw. Muster auf der Oberfläche zu schaffen. In einem Sockel, auf dem Bernstein befestigt ist, können beispielsweise Löcher in einer erforderlichen Größe und Menge eingebracht werden und eine Lichtquelle kann hinter dem Holzsockel oder auch in diesen Löchern installiert werden. Das Licht gelangt dann durch die Löcher im Holzsockel und durch den Bernstein, sodass hier eine Durchlichtbeleuchtung vorgenommen wird.

Weiterhin wäre es auch möglich, Bernsteinplatten herzustellen, was beispielsweise durch Pressen von Naturbernstein in verschiedenen Größen sowie von Bernsteinkrümeln und Staub hergestellt werden kann.

Bernsteinplatten können durch Pressen von Naturbernstein in verschiedenen Größen sowie von Bernsteinspänen und Staub hergestellt werden. Ein Verfahren zur Herstellung eines Bernsteinformprodukts beginnt bevorzugt mit dem Einfüllen von (insbesondere gereinigtem) Bernstein oder Bernsteinschnitzeln in die Form, dann wird die Form zusammen mit Bernstein erhitzt und es wird bevorzugt der Pressvorgang durchgeführt.

An diesen Prozess schließt sich bevorzugt ein Abkühlen des Bernsteins, optional mit seiner Verarbeitung (Mahlen, Schleifen, Polieren) an. Diese Lösung kann in unterschiedlichen Aufenthaltsräumen Anwendung finden,

Bernsteinplatten können in den hier genannten Aufenthaltsräumen oder auch in verschiedene Saunen, Bäder, Hammam und Bernsteinräumen als Verkleidungsmaterial zur Dekoration von Wänden, Decken, Böden und Sitzen verwendet werden. Sie können sich erwärmen und beleuchten.

Bei einem erfindungsgemäßen Verfahren zur Herstellung eines Bernsteinformprodukts wird zunächst Bernstein und insbesondere gereinigter Bernstein oder Bernsteinkrümel in eine Form eingefüllt. Anschließend wird die Form gemeinsam mit dem Bernstein erhitzt werden Bei einem weiteren optionalen Verfahrensschritt wird ein Pressvorgang durchgeführt. In einem weiteren Verfahrensschritt wird anschließend der Bernstein abgekühlt werden und/ oder es kann eine Verarbeitung oder Bearbeitung des Bernsteins erfolgen (Fräsen, Schleifen oder Polieren).

Bevorzugt wird das Bernsteinprodukt in dem Aufenthaltsraum angebracht und/oder befestigt

Die Anmelderin behält sich daher vor Schutz zu beanspruchen für einen betretbaren Aufenthaltsraum für Personen mit wenigstens einer Seitenwandung und mit wenigstens einer Decke, wobei wenigstens an der Seitenwandung oder an der Decke wenigstens eine Bernsteinplatte angeordnet ist. Insbesondere ist diese Bernsteinplatte nach dem oben beschriebenen Verfahren gefertigt.

Weiterhin wäre es auch denkbar, dass der Aufenthaltsraum vollständig mit den Stückkörpern und insbesondere mit Bernstein bedeckt ist. Dabei können insbesondere sämtliche Wände und auch die Decke mit Bernstein bedeckt sein. Daneben können jedoch zusätzlich zu den vollständig bedeckten Wänden und Decken Fenster innerhalb des Raumes angeordnet sein. Daneben können, wie oben erwähnt auch Heizelemente vorgesehen sein. Bei den Räumen kann es sich, wie oben erwähnt beispielsweise um Saunen oder Bernsteinzimmer handeln. Wie oben erwähnt, kann auch der Boden des Aufenthaltsraums mit Bernstein bedeckt sein. Dabei können die Bernsteine auch lose auf dem Boden angeordnet sein bzw. auf dem Boden verstreut sein. Wie oben erwähnt, kann hier auch eine Befestigung mit ökologischen Klebstoffen erfolgen. Allgemein ist auch hier wieder eine Befestigung mit einem Drahtelement oder mittels eines Klebstoffes möglich. Auf die gleiche Weise kann auch ein Träger wie etwa eine Bernsteinplatte befestigt werden.

Bei einer weiteren vorteilhaften Ausführungsform weist der Aufenthaltsraum Sitz- und/oder Liegemöglichkeiten für Personen auf, insbesondere Stühle, Bänke oder Liegen.

Falls das oben erwähnte Netzelement und/oder ein Träger mit daran befestigten Stückkörpern. Insbesondere Bernsteinen zum Befestigen der Stückkörper verwendet wird, kann dieses beispielsweise an Einrichtungsgegenständen wie etwa einer in dem Aufenthaltsraum befindlichen Bank angeordnet sein. So könnte das Netz unter einer Konstruktion einer Bank befestigt sein. Auch könnte das Netzelement unter einer Bank oder auch hinter oder an einer perforierten Wandung befestigt sein.

Daneben könnte das Netzelement auch an den Wänden des Aufenthaltsraumes oder auf dem Boden befestigt werden.

Das mit Bernstein gefüllte Netzelement wird bevorzugt in eine Zelle und/oder einen Abschnitt eingebaut, auf dem/der eine Heizplatte oder allgemein eine Heizeinrichtung angeordnet und insbesondere ebenfalls befestigt ist. Daneben ist es auch möglich, dass sich ein Heizelement und/oder eine Heizplatte innerhalb des Netzelements befindet. Es können auch mehrere Heizelemente vorgesehen, sowohl innerhalb als auch außerhalb des Netzelements.

Beim Ersetzen / Bedienung des Systems kann der Bernstein-Netzkasten leicht entfernt und durch einen neuen ersetzt werden. Die Größen des Netzelements können je nach Größe des Zimmers variieren.

Weitere Vorteile und Ausführungsformen ergeben sich aus der beigefügten Zeichnung.

Darin zeigen:
- Fig. 1: Eine erste Ansicht eines an einem Wand- oder Deckenelement angeordneten Stückkörpers;
- Fig. 2: Eine Seitenansicht der in Figur 1 gezeigten Darstellung;
- Fig. 3: Eine weitere Seitenansicht der in Figur 1 gezeigten Darstellung;
- Fig. 4: Eine Ausgestaltung der Verwendung einer netzartigen Struktur zum Befestigen der Stückkörper;
- Fig. 5: Eine Seitenansicht der in Figur 4 gezeigten Ausgestaltung;
- Fig. 6: Eine Darstellung zur Veranschaulichung einer Beleuchtung der Stückkörper; und
- Fig. 7a-c: Drei Darstellungen zur Veranschaulichung der Befestigung eines Netzelements.

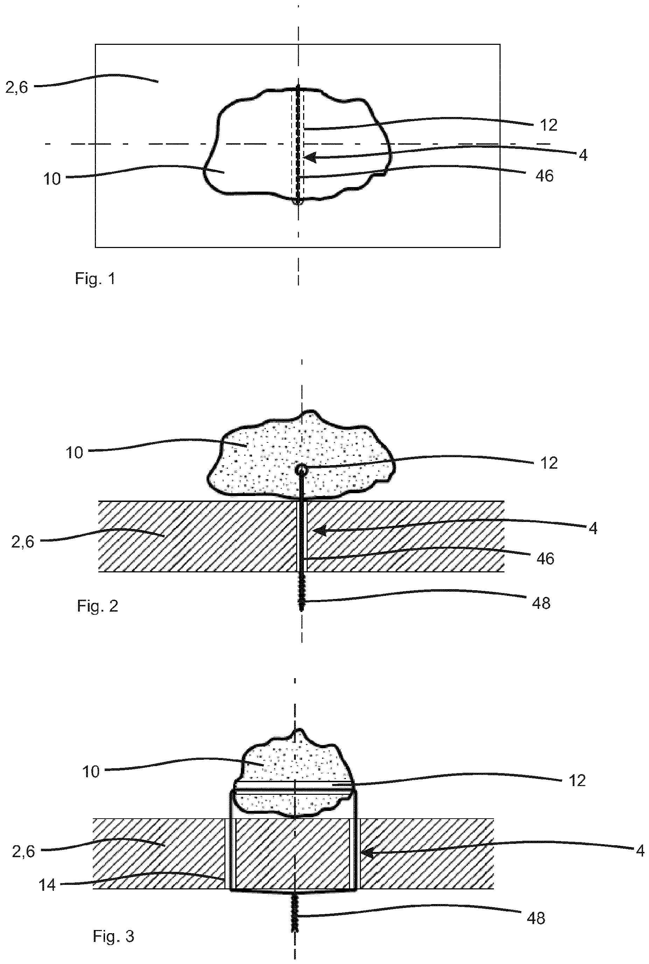

Figur 1 zeigt eine Draufsicht auf eine erfindungsgemäße Anordnung eines Stückkörpers 10 an einem Wand- oder Deckenelement 2, 6. Dabei ist in dem Stückkörper eine Bohrung 12 angeordnet, durch welche hindurch ein Drahtband oder kabelartiges Element geführt wird, welches zur Befestigung des Stückkörpers dient.

Figur 2 zeigt eine Seitendarstellung der in Figur 1 gezeigten Darstellung. Man erkennt hier wiederum das drahtartige Element, das durch eine Öffnung 12 des Stückkörpers 10 geführt ist. In dem Wand- oder Deckenelement 2, 6 ist ebenfalls eine Bohrung bzw. ein Loch vorgesehen, durch welches hindurch das drahtartige Element 46 geführt wird. Das Bezugszeichen 48 kennzeichnet eine Befestigung, mit der die Endabschnitte des Drahtelements aneinander befestigt werden. Insbesondere werden sie bei der in Figur 2 gezeigten Darstellung miteinander verdrillt.

Figur 3 zeigt eine weitere Seitenansicht der in Figur 1 gezeigten Ausgestaltung. Man erkennt hier, dass durch die Öffnung 12 das Drahtelement 46 geführt ist und die beiden Endabschnitte wiederum durch die Öffnungen 14 und 16, die sich in dem Wand- oder Deckenelement 2, 6 befinden, geführt sind. Auf der Unterseite des Wand- oder Deckenelements sind die Enden des drahtartigen Elements 46 miteinander verdrillt, um so einen sicheren Halt des Stückkörpers an dem Wandelement zu erreichen.

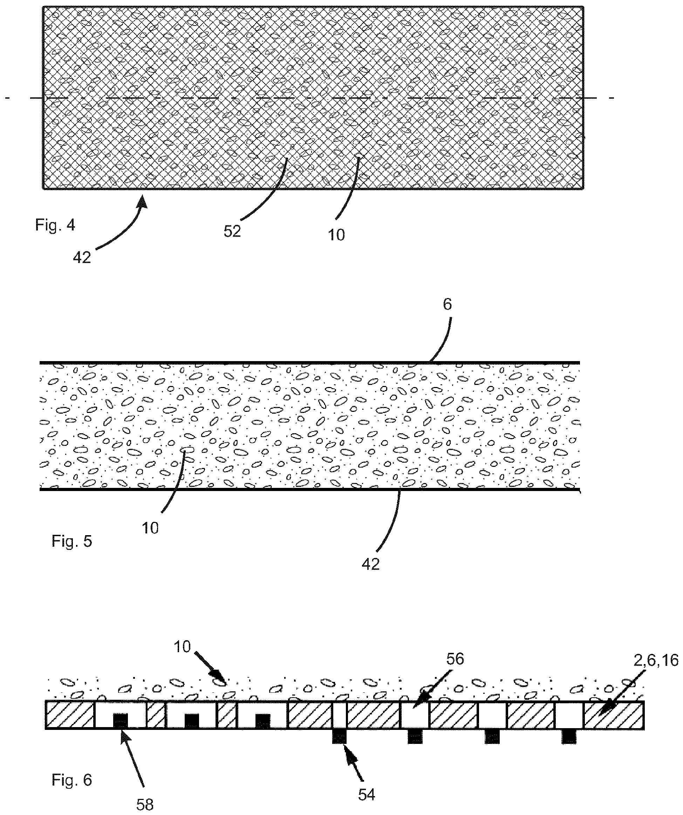

Figur 4 zeigt eine weitere Ausgestaltung einer erfindungsgemäßen Befestigung. Hier ist ein Netz 42 vorgesehen, welches zum Rückhalten der Stückkörper 10 dient. Zu diesem Zweck weist dieses Netz Maschen 52 auf, die jedoch jeweils kleiner sind als die zurückzuhaltenden Stückkörper.

Figur 5 zeigt eine Seitenansicht der in Figur 4 gezeigten Befestigung. Hier ist eine Vielzahl von Stückkörpern 10 vorgesehen, die zwischen dem Wand- oder Deckenelement 2, 6 und dem Netzelement 42 angeordnet sind. Man erkennt, dass die Stückkörper in mehreren Schichten in den gebildeten Zwischenraum eingebracht sind. Auf diese Weise kann eine insgesamt geschlossen wirkende Schicht von Stückkörpern erreicht werden.

Figur 6 zeigt eine Ausgestaltung der erfindungsgemäßen Befestigung unter Berücksichtigung einer Beleuchtung. Hier ist in dem Wand- oder Deckenelement oder auch in einem zusätzlichen Panel 16 eine Vielzahl von Öffnungen 56 vorgesehen. Diese Öffnungen dienen jeweils zum Durchtritt von Licht von Leuchtkörpern oder Lichtquellen 54 und 58. Dabei ist es möglich, dass die Lichtquellen 58 selbst in den genannten Öffnungen angeordnet sind oder auch dahinter. Auf diese Weise kann eine Durchlichtbeleuchtung der Stückkörper vorgenommen werden.

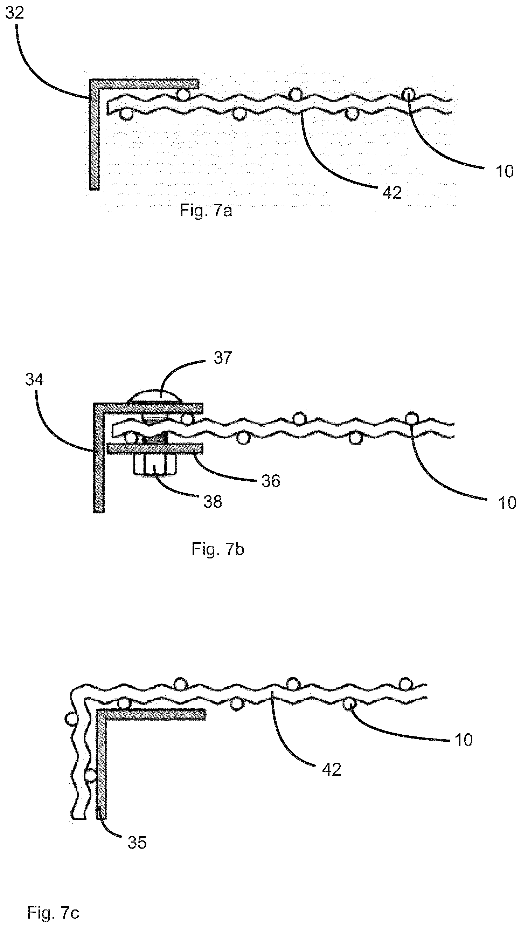

Die Figuren 7a - 7c zeigen drei Möglichkeiten der Befestigung eines Netzelements 42. Bei der in Fig. 7a gezeigten Vorgehensweise ist ein Träger 32, beispielsweise ein Metallträger oder Holzträger vorgesehen, an dem das Netzelement befestigt ist, was beispielsweise durch Schrauben erfolgen kann. Auch ist es möglich dass der Träger das Netzelement an einem anderen Gegenstand (nicht gezeigt) festklemmt.

Bei der in Fig. 7b gezeigten Darstellung ist neben einem Träger 34 auch ein Halteelement 36 vorgesehen. Das Netzelement 42 ist zwischen diesem Träger 34 und dem Halteelement 36 eingeklemmt, wobei hierzu eine Schraube 37 vorgesehen ist, auf welche eine Schraubenmutter 38 aufgeschraubt ist.

Bei der in Fig. 7c gezeigten Ausgestaltung ist ein Stützträger 35 vorgesehen, der hier als Kantenelement ausgeführt ist, wobei das Netz 42 um eine Kante 35a dieses Stützträgers angeordnet ist. Damit wird bei dieser Gestaltung das Netzelement um 90° gekrümmt. Auch der Stützträger kann dabei das Netzelement gegen ein weiteres Element, wie etwa eine Wandung des Aufenthaltsraumes drücken.

Daneben können auch mehrere Netzelemente verwendet werden und/oder die Aufhängung mehrerer Netzelemente in der oben genannten Art und Weise erfolgen.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 10: Stückkörper
- 12: Bohrung
- 14: Öffnung
- 16: Öffnung
- 32, 34: Träger
- 35: Stützträger
- 36: Halteelement
- 37: Schraube
- 38: Schraubenmutter
- 42: Netzelement
- 46: drahtartiges Element
- 52: Maschen
- 54: Lichtquellen
- 56: Öffnungen
- 58: Befestigung
- 58: Lichtquellen
- 2, 6: Wand- oder Deckenelement

## Patentansprüche

1. Betretbarer Aufenthaltsraum für Personen mit wenigstens einer Seitenwandung (2) und mit wenigstens einer Decke (6), wobei wenigstens an der Seitenwandung (2) oder an der Decke (6) eine Vielzahl von Stückkörpern (10) angeordnet ist, wobei sich diese Stückkörper (10) hinsichtlich ihrer äußeren Gestalt voneinander unterscheiden und wobei diese Stückkörper (10) wenigstens abschnittsweise eine nichtebene Oberflächengestalt aufweisen, wobei diese Stückkörper (10) mittels wenigstens einer Befestigungseinrichtung (4) an der Seitenwandung oder der Decke befestigt sind, **dadurch gekennzeichnet, dass**
die Befestigungseinrichtung (4) ein Netzelement (42) aufweisen, welches Netzmaschen (44) aufweist, die derart dimensioniert sind dass die Stückkörper nicht durch diese Netzmaschen (44) treten können und/oder die Befestigungseinrichtung (4) ein drahtartiges Element (46) aufweist, welches durch eine durch den Stückkörper (10) hindurch verlaufende Öffnung (12) geführt ist und/oder die Stückkörper mittels eines Klebstoffes an den Seitenwand oder der Decke befestigt ist.

2. Betretbarer Aufenthaltsraum (1) nach nach Anspruch 1,
**dadurch gekennzeichnet, dass**
Stückkörper Bernsteine (10) sein.

3. Betretbarer Aufenthaltsraum (1) nach wenigstens einem der vorangegangenen Ansprüche,
**dadurch gekennzeichnet, dass**
der Aufenthaltsraum aus einer Gruppe von Aufenthaltsräumen ausgewählt ist, welche Saunen, Ruheräume in Wellnessbereichen, Dampfbäder, Schwimmbäder, Infrarotkabinen, Bernsteinzimmer und dergleichen enthält.

4. Betretbarer Aufenthaltsraum (1) nach wenigstens einem der vorangegangenen Ansprüche,
**dadurch gekennzeichnet, dass**
die Befestigungseinrichtung ein Material aufweist, welches aus einer Gruppe von Materialien ausgewählt ist, welche Kupfer, Silber, Gold, Platin und dergleichen enthält.

5. Betretbarer Aufenthaltsraum (1) nach wenigstens einem der vorangegangenen Ansprüche,
**dadurch gekennzeichnet, dass**
das Netzelement (42) und/oder das Drahtelement (46) aus einem Material hergestellt ist, welches aus einer Gruppe von Materialien ausgewählt ist, welche Kupfer, Silber, Gold, Platin und dergleichen enthält.

6. Betretbarer Aufenthaltsraum (1) nach wenigstens einem der vorangegangenen Ansprüche,
**dadurch gekennzeichnet, dass**
die Drahtelemente (46) an den Seitenwänden und/oder an der Decke und insbesondere an in den Seitenwänden und/oder in der Decke angebrachten Öffnungen befestigt sind.

7. Betretbarer Aufenthaltsraum (1) nach wenigstens einem der vorangegangenen Ansprüche,
**dadurch gekennzeichnet, dass**
Endabschnitte (46a, 46b) miteinander verbunden sind.

8. Betretbarer Aufenthaltsraum (1) nach wenigstens einem der vorangegangenen Ansprüche,
**dadurch gekennzeichnet, dass**
jeder Stückkörper an wenigstens zwei Punkten an der Wandung oder der Decke befestigt ist.

9. Betretbarer Aufenthaltsraum (1) nach wenigstens einem der vorangegangenen Ansprüche,
**dadurch gekennzeichnet, dass**
die Drahtelemente einen Durchmesser haben, der größer ist als 0,1 mm, bevorzugt 0,2mm und besonders bevorzugt größer als 0,3mm und/oder die Drahtelemente einen Durchmesser haben, der kleiner ist als 10mm, bevorzugt kleiner als 8mm, bevorzugt kleiner als 6mm, bevorzugt kleiner als 5mm, bevorzugt kleiner als 4mm und bevorzugt kleiner als 3mm und besonders bevorzugt kleiner als 2mm.

10. Betretbarer Aufenthaltsraum (1) nach wenigstens einem der vorangegangenen Ansprüche,
**dadurch gekennzeichnet, dass**
das Netzelement (42) an der Decke und/oder an wenigstens einer Wand befestigt ist.

11. Betretbarer Aufenthaltsraum (1) nach wenigstens einem der vorangegangenen Ansprüche,
**dadurch gekennzeichnet, dass**
der Aufenthaltsraum eine Belüftungseinrichtung und/oder ein Belüftungssystem aufweist.

12. Betretbarer Aufenthaltsraum (1) nach wenigstens einem der vorangegangenen Ansprüche,
**dadurch gekennzeichnet, dass**
der Aufenthaltsraum eine Beleuchtung zur Beleuchtung der Stückkörper aufweist.

13. Betretbarer Aufenthaltsraum (1) nach wenigstens einem der vorangegangenen Ansprüche,
**dadurch gekennzeichnet, dass**
der Aufenthaltsraum (1) einen Boden aufweist und bevorzugt auch an diesem Boden Stückkörper angeordnet sind.

14. Betretbarer Aufenthaltsraum (1) nach wenigstens einem der vorangegangenen Ansprüche,
**dadurch gekennzeichnet, dass**
der Aufenthaltsraum eine Heizeinrichtung zur Erwärmung des Aufenthaltsraums aufweist.

15. Betretbarer Aufenthaltsraum (1) nach wenigstens einem der vorangegangenen Ansprüche,
**dadurch gekennzeichnet, dass**
innerhalb des Raums plattenartige Verkleidungselemente vorgesehen sind, wobei diese plattenartigen Verkleidungselemente bevorzugt aus Bernstein gefertigt sind.
